# EUROPEAN PATENT SPECIFICATION

(11) **EP 3 826 026 B1**
(45) Date of publication and mention of the grant of the patent: **17.06.2026**
(21) Application number: 19210986.6
(22) Date of filing: 22.11.2019
(51) Int. Cl.: G16H 20/30

(54) **ROBOTIC DEVICE FOR USE IN NEUROREHABILITATION THERAPY**
ROBOTISCHE VORRICHTUNG ZUR VERWENDUNG IN DER NEUROREHABILITATIONSTHERAPIE
DISPOSITIF ROBOTIQUE POUR UNE UTILISATION DANS UNE THÉRAPIE DE NEURORÉADAPTATION

(43) Date of publication of application: 26.05.2021
(73) Proprietor: Platz, Thomas, 17498 Mesekenhagen OT Gross Karrendorf (DE)
(72) Inventor: Platz, Thomas, 17498 Mesekenhagen OT Gross Karrendorf (DE)
(74) Representative: Gulde & Partner

(56) References cited:
- EP-A1- 2 767 967
- EP-B1- 2 969 079
- WO-A2-01/36051
- WO-A2-2005/074373
- JP-A- 2016 080 671
- KR-B1- 101 180 087
- US-A1- 2012 190 505
- US-A1- 2014 347 392
- US-A1- 2015 039 106
- US-A1- 2015 196 804
- US-A1- 2017 368 413
- US-A1- 2018 110 669
- US-A9- 2017 151 500
- REHA TECHNOLOGY AG: "Armotion User Manual", 1 December 2014 (2014-12-01), XP055690342, Retrieved from the Internet <URL:https://fccid.io/2AF6XRTAM1000/User-Manual/User-Manual-2948872.pdf> [retrieved on 20200429]

## Description

The present invention refers to a robotic device for use in neurorehabilitation therapy.

### TECHNOLOGICAL BACKGROUND AND PRIOR ART

Therapeutic training methods aiding recovery from a nervous system injury (e.g. for stroke patients) have progressed steadily over the past decades. In particular, evidence-based therapeutic methods can achieve clinically relevant improvements of dysfunctions due to brain or spinal cord damages, reduction of restrictions through disabilities as well as social participation and inclusion. Such methods can be summarized under the generic term evidence-based neurorehabilitation.

Repetitive training units constitute key elements in neurorehabilitation, which require time and cost intensive therapeutic assistance. Several supporting technical devices are available to the therapist. Such technical devices may comprise electro-mechanic robots, which have been developed for upper limb-rehabilitation (Mehrholz et al., 2018) and gait-rehabilitation (Mehrholz et al., 2017). Another promising approach provides the use of virtual reality applications, which have been recently investigated (Laver et al., 2017).

Reha Technology Ag: "Armotion User Manual", 1 December 2014 (2014-12-01), discloses a robotic device called "The Armotion", an exoskeleton-like robot, for the treatment of severe and moderate neurological and orthopaedic dysfunction of the upper extremity. WO 01/36051 A2 discloses a portable, self-learning adaptive weight bearing monitoring system for personal use during rehabilitation of neurological disorders and orthopedic lower limb injuries, including a flexible insole or pad which includes at least one pressure and/or force sensor that measures the weight force applied to at least two monitored locations of at least one of the patient's limbs.

Most technical approaches aim a one-dimensional support function in a human-to-human therapy. That is, the support function is limited to predetermined and standardized training programs, which have to be performed by the patient by means of the technical (robotic) device, eventually in permanent assistance of the therapist. However, these approaches are not necessarily suitable for an autonomous or semi-autonomous robot-to-human therapy program, which comprise socially interactive components in addition to therapy related training specifications. The socially interactive components must be implemented in the training method to improve the therapeutic efficacy.

### DESCRIPTION OF THE INVENTION

The objective of the invention is solved and the disadvantages of the prior art are overcome, at least partially, by the robotic device according to the appended claims. The dependent claims are directed to preferred embodiments of the present disclosure.

An aspect of the disclosure relates to a robotic device for use in neurorehabilitation therapy. Therein, the robotic device of the present disclosure comprises a human-machine-interface, HMI, configured for allowing human-machine-interaction based on active user inputs of a patient and/or a therapist and based on patient surveillance, i.e., based on monitoring a patient. Particularly, the HMI comprises a sensor system that is configured to detect a motion of a patient. Therein, the motion of the patient refers to the motion of the patient as a whole or to the motion of a part of the patient. Further, the patient motion refers to the process of motion or to one or more motion target states achieved by the motion. The HMI further comprises at least one of an input device that is configured for receiving user input, i.e., input that is actively input by a patient and/or therapist for intentionally controlling the robotic device. Therein, the input device is installed at the robotic device. Additionally, the robotic device comprises a wireless communication interface that is configured to receive user input from a workstation, a mobile device, and/or a local network. In other words, the user input of a patient and/or a therapist is also received remotely from an input mean that is wirelessly connected to the robotic device. The robotic device further comprises an output system that is configured to output instructions and/or feedback to the patient. The output system allows for outputting various content to the patient using different output channels, e.g., in the form of audio, video and/or haptic signals.

The robotic device of the present disclosure further comprises a database storing a plurality of first datasets and a plurality of second datasets. In other words, the database is accessible by the robotic device in order to load a first dataset and/or a second dataset, or parts of a first dataset and/or a second dataset into a processing unit, e.g., a CPU, of the robotic device. According to the present disclosure each first dataset corresponds to a specific neurorehabilitation therapy and comprises first information related to a patient task during the specific neurorehabilitation therapy and comprising instruction information on the task execution to be performed by the patient and further comprising recognition information for detection of the task execution and reference information defining a therapeutic task execution. In other words, the first datasets comprise therapy-specific information for a plurality of different neurorehabilitation therapies. Further each second dataset corresponds to a patient and comprises patient characteristics related to the patient's anatomy, physiology and/or cognitive abilities. In other words, the second datasets comprise patient-specific information for a plurality of patients and/or for a plurality of patient classes (patient categories).

The robotic device of the present disclosure further comprises a processing unit that is functionally connected to the HMI, the output system and the database and is configured to control each of the HMI, the output system and the database. Particularly, the processing unit is configured to receive, via the HMI, a first user input choosing a neurorehabilitation therapy to be performed. Preferably the first user input is received via the input device or, via the wireless communication interface, a connected input means. Further, the first user input may be prompted by a graphical user interface showing a plurality of available neurorehabilitation therapies to a user for selecting a specific neurorehabilitation therapy. Therein, the neurorehabilitation therapies are indicated by a name of the therapy, a symbol, or the like.

The processing unit is further configured to receive, via the HMI, a second user input that is specifying a patient and/or a patient characteristic. Preferably the second user input is received via the input device or, via the wireless communication interface, a connected input means. Further, the second user input may be prompted by a graphical user interface showing a plurality of available patients or patient classes to a user for selecting a specific patient or patient class. Therein, the patients may be indicated by a name and/or a picture of the patient and the patient classes may be indicated by a denomination of the patient class.

The processing unit is further configured to load, from the database, a first dataset based on the first user input. In other words, the processing unit is configured to load a first dataset that corresponds to the neurorehabilitation therapy specified by the first user input. Further, the processing unit is configured to load, from the database, a second dataset based on the second user input. In other words, the processing unit is configured to load a second dataset that corresponds to the patient or patient class specified by the second user input.

The processing unit is further configured to generate personalized instructions for the patient based on the first information of the loaded first dataset and the patient characteristics of the loaded second dataset, wherein the personalized instructions comprise instruction information selected from the instruction information of the first dataset based on the patient characteristics of the second dataset. In other words, the processing unit is configured to use the therapy-specific information of the first dataset in combination with the patient (class)-specific information of the second dataset in order to generate instructions that are personalized for a patient. Therein, the processing unit is preferably configured to use patientunspecific information on a certain neurorehabilitation therapy from the loaded first dataset and therapy-unspecific information on a certain patient (class) from the loaded second dataset for determining personalized instructions for a patient. Embodiments for generating such personalized instructions are described in more detail below. Therein, the personalization of the instructions may refer to the content of the instructions or to the outputting of the instructions. The processing unit is further configured to generate the personalized instructions for the patient based on the instruction information of the loaded first dataset and the patient characteristics of the loaded second dataset. In other words, the processing unit uses the loaded patient characteristics to select the instruction information according to the patient's needs and therefore generates the personalized instructions. Therein the instruction information may comprise, e.g. audio files and/or video files related to the execution task.

The processing unit is further configured to output, via the output system, the personalized instructions to the patient. In other words, the processing unit is configured to control the output system to output the generated personalized instructions to the patient. Therein, the output system may output the personalized instructions with personalized content via one or more output means, e.g., via a display and/or a speaker. Further, the output system may output the personalized instructions via one or more personalized output means, i.e., via an output means selected based on the patient characteristics of the loaded second dataset.

The robotic device of the present invention thus advantageously allows for providing a personalized neurorehabilitation therapy to a patient by using a combination of therapy-specific data and patient-specific data. In other words, the robotic device of the present invention accesses a database with such information in an advantageous manner in order to improve the social interaction between the robotic device and the patient. Indeed, rehabilitation patients value personal interaction high and at times beyond the actual contents of the prescribed therapy (Peiris et al., 2012). It was shown that stroke survivors fail to use rehabilitation technology in self-directed therapy programmes void of personalized social interaction (D-Silva et al., 2018). By combining effective treatment strategies with personalized social interaction the robotic device of the disclosure thus provides a tool for an automated and highly effective neurorehabilitation therapy.

Further, the processing unit is also configured to adapt the first information of the loaded first dataset based on the patient characteristics by adapting the recognition information of the loaded first dataset with regard to information about the patient lodged in the patient characteristics. Further, the processing unit is also configured to control the sensor system to detect the task execution of the patient based on the adapted recognition information; and to generate deviation information based on the detected task execution and on the loaded first dataset; and to generate personalized feedback for the patient based on the deviation information and the patient characteristics of the loaded second dataset; and to output, via the output system, the personalized feedback to the patient.

In a preferred embodiment of the present disclosure, the processing unit of the robotic device is further configured to control the sensor system to detect a task execution of the patient based on the loaded first dataset. In other words, the processing unit may use the therapy-specific information of the loaded first dataset in order to actuate and/or adjust the sensor system to detect a task execution corresponding to the specific neurorehabilitation therapy of the loaded first dataset. Therein the sensor system may detect the task execution via one or more sensor devices, e.g., an image sensor, an audio sensor, a touch sensitive display and/or a temperature sensor.

Subsequently, the processing unit may be configured to generate deviation information based on the detected task execution and the loaded first dataset. That is, the processing unit may be configured to compare the detected data of the task execution with the pre-set data of the loaded first dataset, then evaluate the deviation derived from the comparison according to the loaded first dataset and generate said deviation information.

Further, the processing unit may generate personalized feedback for the patient based on the deviation information and the patient characteristics of the loaded second dataset. In other words, the processing unit may use the patient characteristics of the second dataset to convert the deviation information into a personalized feedback, e.g., into a video file for a patient with language or hearing deficits or an audio file for a patient with visual impairment. Furthermore, the processing unit may be configured to output, via the output system, the personalized feedback to the patient. In other words, the processing unit may be configured to control the output system to output the generated personalized feedback to the patient. Therein, the output system may output the personalized feedback with personalized content via one or more output means, e.g., via a display and/or a speaker. Further, the output system may output the personalized feedback via one or more personalized output means, i.e., via an output means selected based on the patient characteristics of the loaded second dataset.

In another preferred embodiment of the present disclosure, the first information of the first dataset comprises recognition information for detection of the task execution and reference information defining a therapeutic task execution. In that context the processing unit may be further configured to control the sensor system to detect the task execution based on the recognition information of the loaded first dataset. In other words, the processing unit may use the therapy specific recognition information of the loaded first dataset in order to actuate and/or adjust the sensor system to detect a task execution. For example, the recognition information may comprise a plurality of sensor-specific machine codes that can be used by the processing unit to actuate and/or to adjust the sensor system for detection of the task execution. The processing unit may be further configured to temporarily store the detected data of the task execution in a data buffer. Therein the sensor system may detect the task execution via one or more sensor devices, e.g., an image sensor, an audio sensor, a touch sensitive display and/or a temperature sensor.

Further, the processing unit may be configured to generate deviation information based on the detected task execution, on the reference information of the loaded first dataset, and on the second dataset. In other words, the processing unit may be configured to compare the detected data of the task execution with the pre-set data of the reference information, then evaluate the deviation derived from the comparison according to the reference information and generate said deviation information.

As set forth above, the processing unit of the robotic device is configured to adapt the first information of the loaded first dataset based on the patient characteristics of the loaded second dataset. That is, the adapted first information comprises adapted recognition information and the processing unit is further configured to control the sensor system to detect the task execution based on the adapted recognition information. In other words, the processing unit is configured to adjust the (standardized) recognition information with regard to information about e.g. limb size or restricted arm posture of the patient lodged in the patient characteristics of the second dataset. That is, the adapted recognition information improves the sensory detection of the task execution. Moreover, in a preferred embodiment the adapted first information may comprise adapted reference information and the processing unit may be configured to generate deviation information based on the detected task execution and the adapted reference information. In other words, the processing unit is configured to adjust the (standardized) reference information with regard to information about e.g. limb size or restricted arm posture of the patient lodged in the patient characteristics of the second dataset. That is, the adapted reference information improves the quality of the data evaluation comprised in the deviation information.

In another embodiment of the present disclosure, the patient task may constitute a patient motion, e.g. lifting or bending of the patient's limb, rotation of the patient's forearm. A patient motion may be understood as isolated physical exercise to be performed by the patient. In that context, the first information of the first dataset may be movement information, which comprises the recognition information related to motion detection, the reference information defining a therapeutic motion sequence and the task execution related to a motion sequence performed by the patient and/or to a motion result achieved by the patient.

The processing unit of the robotic device is further configured to adapt the movement information of the loaded first dataset based on the patient characteristics of the loaded second dataset. The adapted movement information may comprise adapted recognition information. Further, the processing unit may be configured to control the sensor system to detect the motion sequence of the patient based on the adapted recognition information. Furthermore, the adapted movement information comprises adapted reference information. The processing unit may be further configured to generate deviation information based on the detected motion sequence and the adapted reference information.

Preferably, each of the recognition information and the reference information comprises joint information defining a spatial relationship of at least three target points in a coordinate system and sequence information defining motion of the at least three target points. The processing unit may be further configured to control the sensor system to locate the at least three target points on the body of the patient based on the joint information and to detect motion of the located target points. Preferably, the sensor system of the robotic device comprises an image sensor configured to detect at least three target points on the body of the patient.

Furthermore, processing unit may be configured to compare the detected motion of the located target points with the sequence information of the loaded first dataset and to generate the deviation information based on the comparison.

In another embodiment, the sensor system comprises an image sensor configured to detect at least one target point. The target point to be detected by the image sensor may be located on the patients body in case of monitoring a process of motion or it may be located in an external area in case of monitoring motion target states achieved by the motion. Said external area may be an area (e.g. on a table) in close vicinity of the patient and in close vicinity of the robotic device. That is, the target points to be detected may be located on said area (on a table) for monitoring motion target states achieved by a motion, e.g. monitoring the stacking of objects on a table. The sensor system may further comprise a touch sensitive display configured to receive a haptic user input.

According to one embodiment, the input device comprises an acoustic sensor configured to detect auditory user input. In other words, the user (e.g. the therapist) may select a specific therapy or patient by pronouncing the therapy type or the patient's name, i.e. pronouncing the first and second user input, and the acoustic sensor may be configured to detect this pronounced auditory (first) user input.

In another preferred embodiment of the present disclosure, the output system of the robotic devise comprises a screen and the processing unit is configured to control the screen to display the personalized instructions and/or the personalized feedback to the patient. The screen controlled by the processing unit may display a text based on the instruction information and the second data set. The screen controlled by the processing unit may also display a motion sequence based on the loaded first dataset. Preferably, the output system further comprises a speaker. The processing unit may further be configured to control the speaker to output the personalized instructions and/or the personalized feedback to the patient.

In a further embodiment of the present disclosure, the patient characteristics comprise first information on a hearing impairment of the patient, second information on a visual impairment of the patient, and/or third information on an attention impairment of the patient. The patient characteristics may further comprise fourth information on a language impairment of the patient.

The processing unit may be further configured to output the personalized instructions and/or the personalized feedback via the screen based on the first information on a hearing impairment. In other words, the first information on a hearing impairment of the patient (e.g. a patient with a hearing deficit) triggers the processing unit to output the personalized instructions and/or the personalized feedback via the screen.

The processing unit may be further configured to output the personalized instructions and/or the personalized feedback via the speaker based on the second information on a visual impairment of the patient. In other words, the second information on a visual impairment of the patient (e.g. a patient with a primary visual or visuospatial disorder) triggers the processing unit to output the personalized instructions and/or the personalized feedback via the speaker. The processing unit may be further configured to output the personalized instructions and/or the personalized feedback with increased intensity via the screen and the speaker based on the third information on an attention impairment of the patient. In other words, the third information on an attention impairment of the patient (e.g. related to a patient with an attention deficit disorder) triggers the processing unit to output the personalized instructions and/or the personalized feedback via the speaker and the screen with increased intensity and/or frequency.

The processing unit may be further configured to output the personalized instructions and/or the personalized feedback via the screen based on the fourth information on a language impairment. In other words, the fourth information on a language impairment of the patient (e.g. related to a patient with a language disorder) triggers the processing unit to output the personalized instructions and/or the personalized feedback via the screen (e.g. as non-verbal information).

In another embodiment of the present disclosure, the output system further comprises a hu-manoid robot with mechanic limbs, wherein the processing unit is configured to select at least one mechanic limb based on the joint data and to actuate the selected at least one mechanic limb based on the sequence data.

In another preferred embodiment of the present disclosure, each of the first data sets and each of the second data sets comprises a header portion. The processing unit may be configured to select a first data set based on a comparison of the first user input with the header portions of the first datasets and a second data set based on a comparison of the second user input with the header portions of the second datasets. In other words, the processing unit may be configured to assign the first user input by comparison with header portions of the first datasets, which are small datalinks, instead of directly assigning the user input to the much larger first datasets.

Furthermore, each of the first data sets and each of the second data sets may comprise a body portion. The body portions of the first datasets may comprise the first information. The body portions of the second datasets may comprise the patient characteristics.

In one embodiment of the present disclosure, the body portion of a second dataset further comprises training history related to a patient associated with the header portion of said second dataset. Preferably, the processing unit is further configured to save progress and/or setbacks in the patient's task execution to generate said training history.

In another embodiment of the present disclosure, the body portions of at least some of the second datasets further comprise machine-human interaction specifications associated with patient-specific machine-human interaction.

The electronic or electric devices and/or any other relevant devices or components according to embodiments of the present disclosure described herein, except those described explicitly as hardware, may be implemented utilizing any suitable hardware, firmware (e.g. an application-specific integrated circuit), software, or a combination of software, firmware, and hardware. For example, the various components of these devices may be formed on one integrated circuit (IC) chip or on separate IC chips. Further, the various components of these devices may be implemented on a flexible printed circuit film, a tape carrier package (TCP), a printed circuit board (PCB), or formed on one substrate. The electrical connections or interconnections described herein may be realized by wires or conducting elements, e.g. on a PCB or another kind of circuit carrier. The conducting elements may comprise metallization, e.g. surface metallization and/or pins, and/or may comprise conductive polymers or ceramics. Further electrical energy might be transmitted via wireless connections, e.g. using electromagnetic radiation and/or light.

Further, the various components of these devices may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random access memory (RAM). The computer program instructions may also be stored in other non-transitory computer readable media such as, for example, a CD-ROM, flash drive, or the like.

Also, a person skilled in the art should recognize that the functionality of various computing devices may be combined or integrated into a single computing device (e.g. processing unit), or the functionality of a particular computing device may be distributed across one or more other computing devices without departing from the scope of the exemplary embodiments of the present disclosure.

Further aspects and preferred embodiments of the present disclosure result from the dependent claims, the drawings and the following description of the drawings. Different disclosed embodiments are advantageously combined with each other if not stated otherwise.

### DESCRIPTION OF THE DRAWINGS

The features of the invention become apparent to those skilled in the art by the detailed description of exemplary embodiments with reference to the attached drawings in which:
- Figure 1: illustrates a schematic set up of the robotic device.
- Figure 2: is a schematic workflow according to a first example patient task;
- Figure 3: is a schematic workflow according to a second example patient task.

### DETAILED DESCRIPTION OF THE INVENTION

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art.

Accordingly, processes, elements, and techniques that are not considered necessary to those having ordinary skill in the art for a complete understanding of the aspects and features of the present invention may not be described.

As used herein, the term "and/or" includes any and all combinations of one or more of the associated listed items. Further, the use of "may" when describing embodiments of the present invention refers to "one or more embodiments of the present invention." In the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. Expressions such as "at least one of," when preceding a list of elements, modify the entire list of elements and do not modify the individual elements of the list.

As used herein, the term "substantially", "about," and similar terms are used as terms of approximation and not as terms of degree, and are intended to account for the inherent deviations in measured or calculated values that would be recognized by those of ordinary skill in the art. Further, if the term "substantially" is used in combination with a feature that could be expressed using a numeric value, the term "substantially" denotes a range of +/- 5% of the value centered on the value.

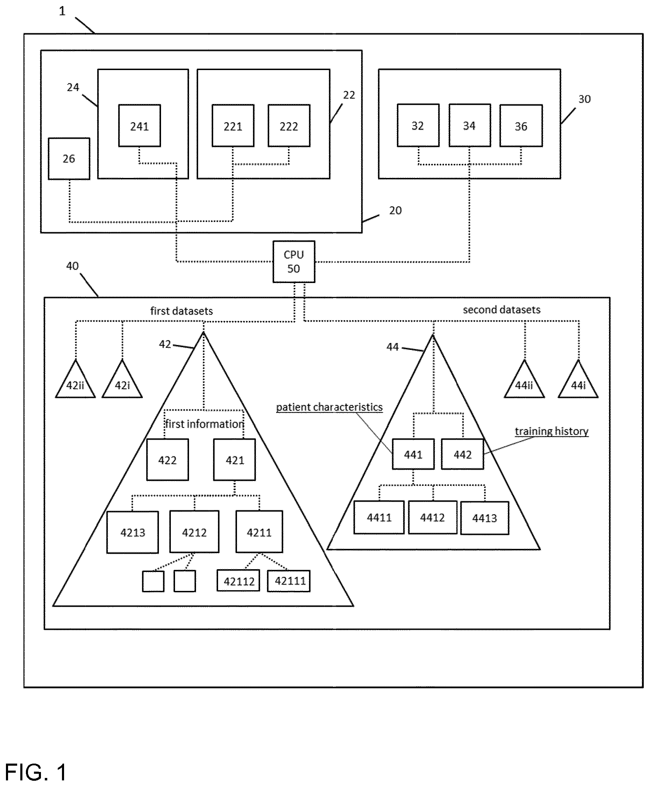

Figure 1 illustrates a schematic set up of the robotic device 1, wherein the data transfer among the components of the robotic device is indicated by means of dotted connections.

The central component of the robotic device 1 is the processing unit 50, which is connected with a human machine interface (HMI) 20, an output system 30 and a database 40.

As shown in figure 1, the HMI 20 has a sensor system 22, which in turn comprises an image sensor 221 configured to detect at least one target point and/or a touch sensitive display 222 configured to receive haptic input. The processing unit 50 is configured to control said sensor system 20 in order to monitor task related parameters such as spatial positioning of one or more target points via the image sensor 222 and/or spatial positioning of a received haptic input via the touch sensitive display 222 based on the recognition information 4211 of the first dataset; and the processing unit 50 is further configured to calculate spatial position change of at least one target point or haptic input, task execution time and/or derive task execution precision based on comparison between the detected parameters and the reference information 4212 of the first dataset 42.

The HMI 20 further has an input device 24, which in turn may comprise an acoustic sensor 241 configured to receive auditory user input. Said auditory user input may be the first user input and/or second user input.

Moreover, the HMI 20 has a wireless communication interface 26 configured to receive user input from a workstation, a mobile device, and/or a local network.

As shown in figure 1 the output system 30 may comprise a screen 32, a speaker 34 and mechanic limbs 36, wherein the processing unit 50 is configured to control the output system 30 to output personalized instructions or personalized feedback to the patient.

The database 40 comprises a series of first datasets 42, 42i, 42ii each related to a specific therapy program, which is accessible via a respective header portion. That is, the processing unit 50 is configured to identify the requested first dataset by comparison of the first user input with only the header portions of the first datasets and assigns and loads the requested first dataset based on that comparison. The header portion of the first datasets may be e.g. the therapy-type, the therapy-duration or the difficulty level of the therapy. Each first dataset further comprises a body portion, which in turn comprises first information 421 related to therapeutic tasks. Each first information 421 (or 422...) related to a therapeutic task comprises recognition information 4211 (4221; not shown in the figure), reference information 4212 (4222; not shown in the figure) and instruction information 4213 (4223; not shown in the figure) in order to pre-set standardized specifications for task-detection, task-analysis and taskinstruction between the robotic device and the patient. For instance the recognition information 4211 may further comprise joint information 42111 and sequence information 42112. That is, depending on the first user input, which might be the therapist choosing therapy A via the HMI 20, the processing unit 50 will compare the header portions corresponding to therapy A to Z with the first user input, identify the header portion related to therapy A and load the respective first dataset of therapy A, respectively the content of the corresponding body portion for subsequent data processing.

The database further comprises a series of second datasets 44, 44i, 44ii each related to a specific patient, which is accessible via a respective header portion. The header portion may be e.g. the patient's name, (national) identification number, health issuance number or the like, suitable to identify a specific person. The header may also be a (standardized) patientgroup, e.g. patients with a hearing loss, language-impaired patients, visually impaired patients or the like, which are characterized by one or more patient characteristics. Each second dataset further comprises a body portion, which in turn comprises patient characteristics 441 related to for instance the patient's anatomy, physiology, psychological and cognitive abilities and behaviour. The patient characteristics may comprise first information on a hearing impairment of the patient 4411, second information on a visual impairment of the patient 4412 and third information on an attention impairment of the patient 4413. That is, depending on a second user input, which might be the therapist choosing patient B via the HMI 20, the processing unit 50 will compare the header portions corresponding to patient A, B, C to Z with the second user input, identify the header portion related to patient B and load the respective second dataset of patient B, respectively the content of the corresponding body portion for subsequent data processing.

In one example the processing unit 50, based on the first and second user input, loads the (standardized) instruction information 4213 of the first dataset 42, which may comprise an audio file and a video file describing instructions on the execution of a task in therapy A, and loads the first information on a hearing impairment of the patient 4411 related to patient B with loss of hearing. Subsequently, the processing unit 50 generates personalized instructions by selecting the video file of the loaded instruction information 4213 based on the first information on a hearing impairment of the patient 4411. The processing unit subsequently controls the output system, respectively the screen 32, to play the video file in order to instruct hearing-impaired patient B to execute the task of therapy A.

The connection between the processing unit 50 and the database 40 may be a wireless local area network (WLAN) connection. The database 40 may be stored on an external computer. Preferably, the database 40 is stored on a (internal) memory unit of the robotic device.

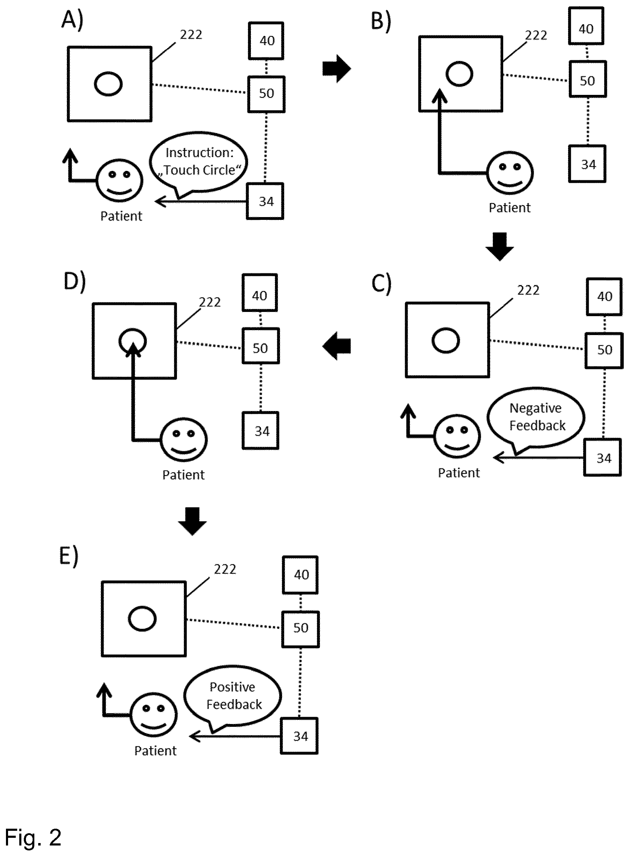

Figure 2 is a workflow according to an example of a patient task, wherein in step A) the patient receives task related personalized instructions via the speaker 34 of the output system. The speaker 34 is controlled by the processing unit 50 that has generated the personalized instructions based on the initial first and second user input (step not shown) and the corresponding first dataset and second dataset loaded from the database 40. Here, the audio file outputted via the speaker 34 contains the instruction to "touch the circle" on a touch sensitive display 222.

In step B) the patient gives the haptic input, respectively touches the touch sensitive display 222 outside the circle. The processing unit 50 is configured to control the touch sensitive display 222 to detect the task execution, respectively the motion target state achieved by the motion, here said haptic input of the patient, based on the recognition information and to generate deviation information based on the spatial positioning of the haptic input and the reference information defining a pre-determined spatial positioning of the haptic input. Subsequently, the processing unit 50 generates a personalised feedback for the patient based on the generated deviation information and the patient characteristics. Depending on the a patient's psychological characteristics (as stored in the body portion of the second dataset comprising patient characteristics 441) the way the negative feedback will be presented needs to be adapted to be in the patient's best interest, i.e. to individually have a positive behaviour shaping quality.

In step C) the processing unit initiates the output of the personalized feedback, here an audio file which contains a negative feedback including motivational comments (e.g. cheering), via the speaker 34 to the patient.

In step D) the patient repeats the task and touches the circle on the touch sensitive screen 222. Again the processing unit controls the touch sensitive display 222 to detect the task execution, generates deviation information on which bases the processing unit 50 generates a personalized feedback.

In step E) the processing unit 50 initiates the output of the personalized feedback, here an audio file which contains a positive feedback including e.g. motivational congratulations, via the speaker 34 to the patient.

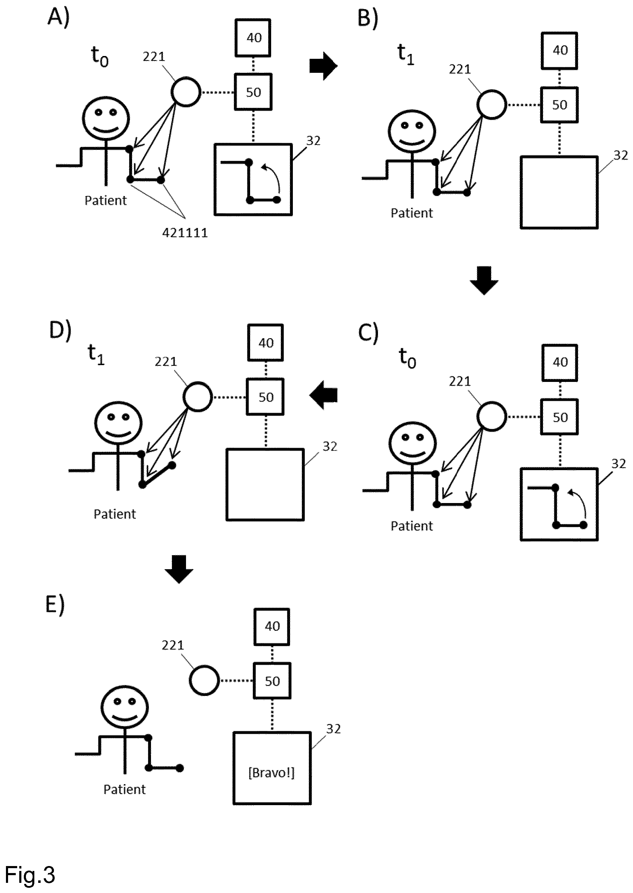

Figure 3 is a workflow according to another example, wherein in step A) the processing unit 50 controls the image sensor 221 to locate three target points 421111 on the arm of the patient based on the joint information 42111 (not shown in the figure, cf. figure 1) defining spatial target points provided by the recognition data 4211 (not shown in the figure, cf. figure 1) of the loaded first dataset and the patient receives task related personalized instructions via the screen 32 of the output system. The screen 32 is controlled by the processing unit 50 that has generated the personalized instructions based on the initial first and second user input (step not shown) and the corresponding first dataset and second dataset loaded from the database 40. Here, the video file outputted via the screen 32 contains the visual instruction to "to bend the arm" according to the reference information of the loaded first dataset containing sequence information defining motion of the at least three target points on a patients arm.

In step B) the patient fails to bend the arm. The processing unit 50 is configured to control the image sensor 221 to detect the task execution, here said bending of the patient's arm, and to generate deviation information based on comparison of the spatial positioning of the target points 421111 at different times (start t₀ and end t₁ of the task execution) and the reference information defining a pre-determined spatial positioning change of the target points. Subsequently, the processing unit 50 generates a personalised feedback for the patient based on the generated deviation information and the patient characteristics.

In step C) the processing unit initiates the output of the personalized feedback, here a video file which shows the bending of an arm, via the screen 32 to the patient.

In step D) the patient repeats the task and bends the arm at a degree. Again the processing unit controls the image sensor to detect the task execution, generates deviation information on which bases the processing unit 50 generates a personalized feedback.

In step E) the processing unit 50 initiates the output of the personalized feedback, here a text file which contains a text message as feedback e.g. motivational congratulations ([Bravo!]), via the screen 32 to the patient. In case of a language-impaired patient such personalized feedback would be presented pictographically (i.e. in a non-verbal manner with "clapping hands") (not shown in figure 3).

### REFERENCE NUMBERS

- 1: robotic device
- 20: human machine interface (HMI)
- 22: sensor system
- 221: image sensor
- 222: acoustic sensor
- 223: touch sensitive display
- 24: wireless communication interface
- 26: input device
- 30: output system
- 32: screen
- 34: speaker
- 36: mechanic limbs
- 40: database
- 42, 42i, 42ii: first dataset(s)
- 421: first information
- 422: movement information
- 4211: recognition information
- 42111: joint information
- 42112: sequence information
- 421111: target points
- 4212: reference information
- 4213: instruction information
- 44, 44i, 44ii: second dataset(s)
- 441: patient characteristics
- 442: training history
- 4411: first information on a hearing impairment of the patient
- 4412: second information on a visual impairment of the patient
- 4413: third information on an attention impairment of the patient
- 50: processing unit (CPU)

## Claims

1. Robotic device for use in neurorehabilitation therapy, the robotic device (1) comprising:
a human-machine-interface, HMI (20), with a sensor system (22) configured to detect a motion of a patient and with at least one of an input device (24) configured for receiving user input and a wireless communication interface (26) configured to receive user input from a workstation, a mobile device, and/or a local network;
an output system (30) configured to output instructions and feedback to the patient;
a database (40) storing a plurality of first datasets (42) and a plurality of second datasets (44), wherein each first dataset (42) corresponds to a specific neurorehabilitation therapy and comprises first information (421) related to a patient task during the specific neurorehabilitation therapy and comprising instruction information (4213) on the task to be performed by the patient and further comprising recognition information (4211) for detection of the task execution and reference information (4212) defining a therapeutic task execution, and wherein each second dataset (44) corresponds to a patient and comprises patient characteristics (441); and
a processing unit (50) that is configured to:
receive, via the HMI (20), a first user input choosing a neurorehabilitation therapy to be performed and a second user input specifying a patient and/or a patient characteristic;
load, from the database (40), a first dataset (42) based on the first user input, and a second dataset (44) based on the second user input;
adapt the first information (421) of the loaded first dataset (42) based on the patient characteristics (441) of the loaded second dataset (44);
generate personalized instructions for the patient based on the first information (421) of the loaded first dataset (42) and the patient characteristics (441) of the loaded second dataset (44), wherein the personalized instructions comprise instruction information selected from the instruction information (4213) of the first dataset (42) based on the patient characteristics (441) of the second dataset (44);
and output, via the output system (30), the personalized instructions to the patient,
adapt the first information (421) of the loaded first dataset (42) based on the patient characteristics (441) by adapting the recognition information (4211) of the loaded first dataset (42) with regard to information about the patient lodged in the patient characteristics (441),
control the sensor system (22) to detect the task execution of the patient based on the adapted recognition information;
generate deviation information based on the detected task execution and on the loaded first dataset (42); and
generate personalized feedback for the patient based on the deviation information and the patient characteristics (441) of the loaded second dataset (44); and
output, via the output system (30), the personalized feedback to the patient.

2. Robotic device according to claim 1, wherein the patient task is a patient motion, the recognition information relates to motion detection, the reference information defines a therapeutic motion sequence and the task execution relates to a motion sequence performed by the patient and/or to a motion result achieved by the patient.

3. Robotic device (1) according to any one of the preceding claims, wherein the sensor system (22) comprises an image sensor (221) configured to detect at least one target point and/or a touch sensitive display (222) configured to receive a haptic user input.

4. Robotic device (1) according to any one of the preceding claims, wherein the input device (24) comprises an acoustic sensor (241) configured to detect auditory user input.

5. Robotic device (1) according to any one of the preceding claims,
wherein the output system (30) comprises a screen (32) and wherein the processing unit (50) is configured to control the screen (32) to display the personalized instructions and/or the personalized feedback to the patient, and/or
wherein the output system (30) comprises a speaker (34) and wherein the processing unit (50) is configured to control the speaker (34) to output the personalized instructions and/or the personalized feedback to the patient.

6. Robotic device (1) according to claim 5,
wherein the patient characteristics (441) comprises first information (4411) on a hearing impairment of the patient, second information (4412) on a visual impairment of the patient, and/or third information (4413) on an attention impairment of the patient, and
wherein the processing unit (50) is further configured to:
output the personalized instructions and/or the personalized feedback via the screen (32) based on the first information (4411), to output the personalized instructions and/or the personalized feedback via the speaker (34) based on the second information (4412), and to output the personalized instructions and/or the personalized feedback with increased intensity via the screen (32) and the speaker (34) based on the third information (4413).

7. Robotic device (1) according to any one of the preceding claims,
wherein each of the first data sets (42) and each of the second data sets (44) comprises a header portion and wherein the processing unit (50) is configured to select a first data set (42,42i,42ii) based on a comparison of the first user input with the header portions of the first datasets (42) and a second data set (44) based on a comparison of the second user input with the header portions of the second datasets (44,44i,44ii).

8. Robotic device (1) according to claim 7, wherein each of the first data sets (42) and each of the second data sets (44) comprises a body portion and wherein the body portions of the first datasets comprises the first information (421) and wherein the body portions of the second datasets comprises the patient characteristics (441).

9. Robotic device (1) according to claim 8, wherein the body portion of a second dataset further comprises training history (442) related to a patient associated with the header portion of said second dataset, and wherein the processing unit (50) is further configured to save progress and/or setbacks in the patient's task execution to generate said training history.

10. Robotic device (1) according to claim 8 or 9, wherein the body portions of at least some of the second datasets further comprises machine-human interaction specifications associated with patient-specific machine-human interaction.

## Patentansprüche

1. Robotische Vorrichtung zur Verwendung in der Neurorehabilitationstherapie, wobei die robotische Vorrichtung (1) Folgendes umfasst:
eine Mensch-Maschine-Schnittstelle, HMI, (20) mit einem Sensorsystem (22), das zum Erfassen einer Bewegung eines Patienten konfiguriert ist, und mit mindestens einer von einer Eingabevorrichtung (24), die zum Empfangen von Benutzereingaben konfiguriert ist, und einer drahtlosen Kommunikationsschnittstelle (26), die zum Empfangen von Benutzereingaben von einer Workstation, einer mobilen Vorrichtung und/oder einem lokalen Netzwerk konfiguriert ist;
ein Ausgabesystem (30), das dazu konfiguriert ist, Anweisungen und Rückmeldungen an den Patienten auszugeben;
eine Datenbank (40), in der mehrere erste Datensätze (42) und mehrere zweite Datensätze (44) gespeichert sind, wobei jeder erste Datensatz (42) einer spezifischen Neurorehabilitationstherapie entspricht und erste Informationen (421) umfasst, die sich auf eine Patientenaufgabe während der spezifischen Neurorehabilitationstherapie beziehen und Anweisungsinformationen (4213) zu der vom Patienten auszuführenden Aufgabe und ferner Erkennungsinformationen (4211) zum Erfassen der Aufgabenausführung und Referenzinformationen (4212) umfassen, die eine therapeutische Aufgabenausführung definieren, und wobei jeder zweite Datensatz (44) einem Patienten entspricht und Patientenmerkmale (441) umfasst; und
eine Verarbeitungseinheit (50), die dazu konfiguriert ist,
über die HMI (20) eine erste Benutzereingabe, mit der eine durchzuführende Neurorehabilitationstherapie ausgewählt wird, und eine zweite Benutzereingabe zu empfangen, mit der ein Patient und/oder ein Patientenmerkmal angegeben werden;
aus der Datenbank (40) einen ersten Datensatz (42) auf der Grundlage der ersten Benutzereingabe und einen zweiten Datensatz (44) auf der Grundlage der zweiten Benutzereingabe zu laden;
die ersten Informationen (421) des geladenen ersten Datensatzes (42) auf der Grundlage der Patientenmerkmale (441) des geladenen zweiten Datensatzes (44) anzupassen;
personalisierte Anweisungen für den Patienten auf der Grundlage der ersten Informationen (421) des geladenen ersten Datensatzes (42) und der Patientenmerkmale (441) des geladenen zweiten Datensatzes (44) zu erzeugen, wobei die personalisierten Anweisungen Anweisungsinformationen umfassen, die aus den Anweisungsinformationen (4213) des ersten Datensatzes (42) auf der Grundlage der Patientenmerkmale (441) des zweiten Datensatzes (44) ausgewählt werden;
und die personalisierten Anweisungen über das Ausgabesystem (30) an den Patienten auszugeben,
die ersten Informationen (421) des geladenen ersten Datensatzes (42) auf der Grundlage der Patientenmerkmale (441) anzupassen, indem die Erkennungsinformationen (4211) des geladenen ersten Datensatzes (42) in Bezug auf in den Patientenmerkmalen (441) hinterlegte Informationen über den Patienten angepasst werden,
das Sensorsystem (22) zu steuern, um die Aufgabenausführung des Patienten auf der Grundlage der angepassten Erkennungsinformationen zu erfassen;
Abweichungsinformationen auf der Grundlage der erfassten Aufgabenausführung und des geladenen ersten Datensatzes (42) zu erzeugen; und
eine personalisierte Rückmeldung für den Patienten auf der Grundlage der Abweichungsinformationen und der Patientenmerkmale (441) des geladenen zweiten Datensatzes (44) zu erzeugen; und
die personalisierte Rückmeldung über das Ausgabesystem (30) an den Patienten auszugeben.

2. Robotische Vorrichtung nach Anspruch 1, wobei die Patientenaufgabe eine Patientenbewegung ist, sich die Erkennungsinformationen auf die Bewegungserfassung beziehen, die Referenzinformationen eine therapeutische Bewegungssequenz definieren und sich die Aufgabenausführung auf eine vom Patienten ausgeführte Bewegungssequenz und/oder auf ein vom Patienten erzieltes Bewegungsergebnis bezieht.

3. Robotische Vorrichtung (1) nach einem der vorstehenden Ansprüche, wobei das Sensorsystem (22) einen Bildsensor (221), der dazu konfiguriert ist, mindestens einen Zielpunkt zu erfassen, und/oder eine berührungsempfindliche Anzeige (222) umfasst, die dazu konfiguriert ist, eine haptische Benutzereingabe zu empfangen.

4. Robotische Vorrichtung (1) nach einem der vorstehenden Ansprüche, wobei die Eingabevorrichtung (24) einen akustischen Sensor (241) umfasst, der dazu konfiguriert ist, akustische Benutzereingaben zu erfassen.

5. Robotische Vorrichtung (1) nach einem der vorstehenden Ansprüche,
wobei das Ausgabesystem (30) einen Bildschirm (32) umfasst und wobei die Verarbeitungseinheit (50) dazu konfiguriert ist, den Bildschirm (32) zu steuern, um dem Patienten die personalisierten Anweisungen und/oder die personalisierte Rückmeldung anzuzeigen, und/oder
wobei das Ausgabesystem (30) einen Lautsprecher (34) umfasst und wobei die Verarbeitungseinheit (50) dazu konfiguriert ist, den Lautsprecher (34) zu steuern, um die personalisierten Anweisungen und/oder die personalisierte Rückmeldung an den Patienten auszugeben.

6. Robotische Vorrichtung (1) nach Anspruch 5,
wobei die Patientenmerkmale (441) erste Informationen (4411) über eine Hörbeeinträchtigung des Patienten, zweite Informationen (4412) über eine Sehbeeinträchtigung des Patienten und/oder dritte Informationen (4413) über eine Aufmerksamkeitsbeeinträchtigung des Patienten umfassen, und
wobei die Verarbeitungseinheit (50) ferner dazu konfiguriert ist,
die personalisierten Anweisungen und/oder die personalisierte Rückmeldung über den Bildschirm (32) auf der Grundlage der ersten Informationen (4411) auszugeben, die personalisierten Anweisungen und/oder die personalisierte Rückmeldung über den Lautsprecher (34) auf der Grundlage der zweiten Informationen (4412) auszugeben und die personalisierten Anweisungen und/oder die personalisierte Rückmeldung mit erhöhter Intensität über den Bildschirm (32) und den Lautsprecher (34) auf der Grundlage der dritten Informationen (4413) auszugeben.

7. Robotische Vorrichtung (1) nach einem der vorstehenden Ansprüche,
wobei jeder der ersten Datensätze (42) und jeder der zweiten Datensätze (44) einen Kopfabschnitt umfassen und wobei die Verarbeitungseinheit (50) dazu konfiguriert ist, einen ersten Datensatz (42, 42i, 42ii) auf der Grundlage eines Vergleichs der ersten Benutzereingabe mit den Kopfabschnitten der ersten Datensätze (42) und einen zweiten Datensatz (44) auf der Grundlage eines Vergleichs der zweiten Benutzereingabe mit den Kopfabschnitten der zweiten Datensätze (44, 44i, 44ii) auszuwählen.

8. Robotische Vorrichtung (1) nach Anspruch 7, wobei jeder der ersten Datensätze (42) und jeder der zweiten Datensätze (44) einen Körperabschnitt umfassen, wobei die Körperabschnitte der ersten Datensätze die ersten Informationen (421) und die Körperabschnitte der zweiten Datensätze die Patientenmerkmale (441) umfassen.

9. Robotische Vorrichtung (1) nach Anspruch 8, wobei der Körperabschnitt eines zweiten Datensatzes ferner eine Trainingshistorie (442) umfasst, die sich auf einen Patienten bezieht, der mit dem Kopfabschnitt des besagten zweiten Datensatzes assoziiert ist, und wobei die Verarbeitungseinheit (50) ferner dazu konfiguriert ist, Fortschritte und/oder Rückschläge bei der Aufgabenausführung des Patienten zu speichern, um die besagte Trainingshistorie zu erzeugen.

10. Robotische Vorrichtung (1) nach Anspruch 8 oder 9, wobei die Körperabschnitte zumindest einiger der zweiten Datensätze ferner Maschinen-Mensch-Interaktionsspezifikationen umfassen, die mit einer patientenspezifischen Maschinen-Mensch-Interaktion assoziiert sind.

## Revendications

1. Dispositif robotique pour une utilisation dans une thérapie de neuroréadaptation, le dispositif robotique (1) comprenant :
une interface homme-machine, IHM (20), avec un système de capteur (22) configuré pour détecter un mouvement d'un patient et avec au moins un d'un dispositif d'entrée (24) configuré pour recevoir une entrée utilisateur et d'une interface de communication sans fil (26) configurée pour recevoir une entrée utilisateur depuis une station de travail, un dispositif mobile et/ou un réseau local ;
un système de sortie (30) configuré pour envoyer des instructions et des retours vers le patient ;
une base de données (40) stockant une pluralité de premiers ensembles de données (42) et une pluralité de seconds ensembles de données (44), dans laquelle chaque premier ensemble de données (42) correspond à une thérapie de neuroréadaptation spécifique et comprend une première information (421) relative à une tâche de patient pendant la thérapie de neuroréadaptation spécifique et comprenant une information d'instruction (4213) sur la tâche à exécuter par le patient et comprenant en outre une information de reconnaissance (4211) pour la détection de l'exécution de tâche et une information de référence (4212) définissant une exécution de tâche thérapeutique, et dans laquelle chaque second ensemble de données (44) correspond à un patient et comprend des caractéristiques de patient (441) ; et
une unité de traitement (50) qui est configurée pour :
recevoir, à travers l'IHM (20), une première entrée utilisateur choisissant une thérapie de neuroréadaptation à exécuter et une seconde entrée utilisateur spécifiant un patient et/ou une caractéristique de patient ;
charger, à partir de la base de données (40), un premier ensemble de données (42) sur la base de la première entrée utilisateur, et un second ensemble de données (44) sur la base de la seconde entrée utilisateur ;
adapter la première information (421) du premier ensemble de données (42) chargé sur la base des caractéristiques de patient (441) du second ensemble de données (44) chargé ;
générer des instructions personnalisées pour le patient sur la base de la première information (421) du premier ensemble de données (42) chargé et des caractéristiques de patient (441) du second ensemble de données (44) chargé, dans lequel les instructions personnalisées comprennent une information d'instruction sélectionnée à partir de l'information d'instruction (4213) du premier ensemble de données (42) sur la base des caractéristiques de patient (441) du second ensemble de données (44) ;
et envoyer, à travers le système de sortie (30), les instructions personnalisées vers le patient,
adapter la première information (421) du premier ensemble de données (42) chargé sur la base des caractéristiques de patient (441) en adaptant l'information de reconnaissance (4211) du premier ensemble de données (42) chargé par rapport à une information sur le patient concerné par les caractéristiques de patient (441),
commander le système de capteur (22) à détecter l'exécution de tâche par le patient sur la base de l'information de reconnaissance adaptée ;
générer une information de déviation sur la base de l'exécution de tâche détectée et du premier ensemble de données (42) chargé ; et
générer un retour personnalisé pour le patient sur la base de l'information de déviation et des caractéristiques de patient (441) du second ensemble de données (44) chargé ; et
envoyer, à travers le système de sortie (30), le retour personnalisé vers le patient.

2. Dispositif robotique selon la revendication 1, dans lequel la tâche de patient est un mouvement du patient, les informations de reconnaissance se rapportent à une détection de mouvement, les informations de référence définissent une séquence de mouvements thérapeutiques et l'exécution de tâche se rapporte à une séquence de mouvements effectués par le patient et/ou à un résultat de mouvement atteint par le patient.

3. Dispositif robotique (1) selon l'une quelconque des revendications précédentes, dans lequel le système de capteur (22) comprend un capteur d'image (221) configuré pour détecter au moins un point cible et/ou un écran tactile sensible (222) configuré pour recevoir une entrée utilisateur haptique.

4. Dispositif robotique (1) selon l'une quelconque des revendications précédentes, dans lequel le dispositif d'entrée (24) comprend un capteur acoustique (241) configuré pour détecter une entrée utilisateur auditive.

5. Dispositif robotique (1) selon l'une quelconque des revendications précédentes,
dans lequel le système de sortie (30) comprend un écran (32) et dans lequel l'unité de traitement (50) est configurée pour commander l'écran (32) à afficher les instructions personnalisées et/ou les retours personnalisés au patient, et/ou
dans lequel le système de sortie (30) comprend un haut-parleur (34) et dans lequel l'unité de traitement (50) est configurée pour commander le haut-parleur (34) à envoyer les instructions personnalisées et/ou les retours personnalisés vers le patient.

6. Dispositif robotique (1) selon la revendication 5,
dans lequel les caractéristiques de patient (441) comprennent une première information (4411) sur une déficience auditive du patient, une deuxième information (4412) sur une déficience visuelle du patient, et/ou une troisième information (4413) sur une déficience de l'attention du patient, et
dans lequel l'unité de traitement (50) est en outre configurée pour :
envoyer les instructions personnalisées et/ou les retours personnalisés à travers l'écran (32) sur la base de la première information (4411), envoyer les instructions personnalisées et/ou les retours personnalisés à travers le haut-parleur (34) sur la base de la deuxième information (4412), et envoyer les instructions personnalisées et/ou les retours personnalisés à une intensité augmentée à travers l'écran (32) et le haut-parleur (34) sur la base de la troisième information (4413).

7. Dispositif robotique (1) selon l'une quelconque des revendications précédentes,
dans lequel chacun des premiers ensembles de données (42) et des seconds ensembles de données (44) comprend une portion de tête et dans lequel l'unité de traitement (50) est configurée pour sélectionner un premier ensemble de données (42, 42i, 42ii) sur la base d'une comparaison de la première entrée utilisateur avec les portions de tête des premiers ensembles de données (42) et un second ensemble de données (44) sur la base d'une comparaison de la seconde entrée utilisateur avec les portions de tête des seconds ensembles de données (44, 44i, 44ii).

8. Dispositif robotique (1) selon la revendication 7, dans lequel chacun des premiers ensembles de données (42) et des seconds ensembles de données (44) comprend une portion de corps et dans lequel les portions de corps des premiers ensembles de données comprennent la première information (421) et dans lequel les portions de corps des seconds ensembles de données comprennent les caractéristiques de patient (441).

9. Dispositif robotique (1) selon la revendication 8, dans lequel la portion de corps d'un second ensemble de données comprend en outre un historique de formation (442) relatif à un patient associé à la portion de tête du second ensemble de données, et dans lequel l'unité de traitement (50) est en outre configurée pour enregistrer des progrès et/ou des reculs pendant l'exécution de tâche du patient afin de générer l'historique de formation.

10. Dispositif robotique (1) selon la revendication 8 ou 9, dans lequel les portions de corps d'au moins certains des seconds ensembles de données comprennent en outre des spécifications d'interaction homme-machine associées à patient-interaction homme-machine spécifique.
